# EUROPEAN PATENT SPECIFICATION

(11) **EP 2 363 703 B1**
(45) Date of publication and mention of the grant of the patent: **07.05.2014**
(21) Application number: 11156839.0
(22) Date of filing: 03.03.2011
(51) Int. Cl.: G01N 3/30, G01N 29/04, G01N 29/24, G01N 29/27

(54) **Method and apparatus for determining the natural frequency of wooden planks**
Methode und Vorrichtung zur Bestimmung der Eigenfrequenz von Holzbrettern
Méthode et appareil pour déterminer la fréquence naturelle de planches de bois

(30) Priority: 03.03.2010 IT VR20100039
(43) Date of publication of application: 07.09.2011
(73) Proprietor: MICROTEC S.r.l., 39042 Bressanone (Bolzano) (IT)
(72) Inventor: Bacher, Martin, 39030, Pfalzen (Bolzano) (IT)
(74) Representative: Ponchiroli, Simone

(56) References cited:
- EP-A1- 1 950 561
- WO-A1-2004/106918
- DE-U1- 9 315 506
- US-B1- 6 347 542
- US-B1- 6 356 846

## Description

This invention relates to a method and an apparatus for determining the natural frequency of wooden planks.

That information is very important when assessing the possible structural uses of a plank. Once the natural frequency of a plank is known, as well as its density, it is possible to determine its modulus of elasticity (using known formulas which therefore are not indicated herein). Determining the modulus of elasticity allows assessment of the possible uses of the plank. Moreover, the greater the modulus of elasticity is, the better the mechanical properties are and therefore the greater the value of the wood.

Consequently, over the years many methods have been developed for assessing the modulus of elasticity, some destructive and some non-destructive.

In the former type of methods, the modulus of elasticity of a batch of planks from the same log is assessed by sacrificing several planks which are tested with traction or bending until they break.

In contrast, determining the modulus of elasticity by measuring the natural frequency belongs to the latter type of methods, which are non-destructive.

The current situation is that it has been known for many years that the natural frequency (or a frequency which is a multiple of it) of a plank may be determined by inducing a vibration in the plank and measuring the frequency of the vibration using suitable detectors. An example of this application is described for example in patent WO 99/44059.

In most cases, the vibration is induced by striking the plank with a hammer, whilst the vibration is detected either using a microphone or using a laser sensor. Moreover, if a microphone is used, it can operate either in contact or at a distance (in this case it is preferably a directional microphone).

Patent US 6347542 B1 teaches that, at the moment of the hammer impact, the microphone should be placed essentially in the middle of the width of the plank and that if the width of the plank varies, then in order to improve performance when using contactless microphones a number of microphones can be arranged in series, whereby recording from the most correctly positioned microphone can be used. Moreover, to guarantee that the plank vibrates in a substantially undisturbed way, during the detection step it is supported by suitable elastic means which do not interfere with the vibration.

Over the years, developments in plants for measuring the natural frequency led to the development of systems able to measure the natural frequency as the planks were fed along a movement path. In this case, both striking of the plank and detection of the vibration with the microphone or the laser detector are carried out while the plank is being fed on a suitable conveyor able to avoid interfering with the vibration.

The detection reading is usually performed by observing the lateral surface of the planks, which is positioned parallel with the movement path.

In particular, when the planks are fed positioned transversally to the movement path (that is to say, alongside each other along their main direction of extension), the detection surface is usually the surface of the end of the plank, that is to say, the smaller surface.

Consequently, since the detector is fixed, and since in order to perform a correct detection reading the plank must be "observed" at least for a predetermined time (the known algorithms for frequency reconstruction require observation to last a predetermined time to guarantee the correctness of the result), the feed speed of the planks along the movement path is limited by the width of the surface examined. Therefore, since the surface is small and its width limited, there is a limit on the feed speed beyond which it is impossible to go.

However, this creates significant disadvantages in more modern plants where optimisation of the various operating stations has allowed very high plank transversal movement speeds to be achieved, which are incompatible with measurement of the natural frequency when the end surface of the plank is small.

Consequently, at present the station for detecting the natural frequency is the bottleneck in wood processing plants.

In this situation, the technical purpose which forms the basis of this invention is to provide a method and an apparatus for determining the natural frequency of wooden planks which overcomes the above-mentioned disadvantages.

In particular, the technical purpose of this invention is to provide a method and an apparatus for determining the natural frequency of wooden planks which allows the natural frequency to be determined by feeding the planks in such a way that they are positioned transversally to the feed path with a feed speed higher than the maximum speed allowed for conventional plants.

The technical purpose specified and the aims indicated are substantially achieved by a method and an apparatus for determining the natural frequency of wooden planks as described in the appended claims.

Further features and the advantages of this invention are more apparent in the detailed description of a preferred, non-limiting embodiment of a method and an apparatus for determining the natural frequency of wooden planks, illustrated in the accompanying drawings, in which:
- Figure 1 is a schematic plan view of an apparatus made according to this invention;
- Figure 2 shows an enlarged detail of the apparatus of Figure 1 during a first step of the method according to this invention;
- Figure 3 shows the detail of Figure 2 during a second operating step of the method according to this invention;
- Figure 4 shows the detail of Figure 2 during a third operating step of the method according to this invention; and
- Figure 5 shows the detail of Figure 2 during a fourth operating step of the method according to this invention.

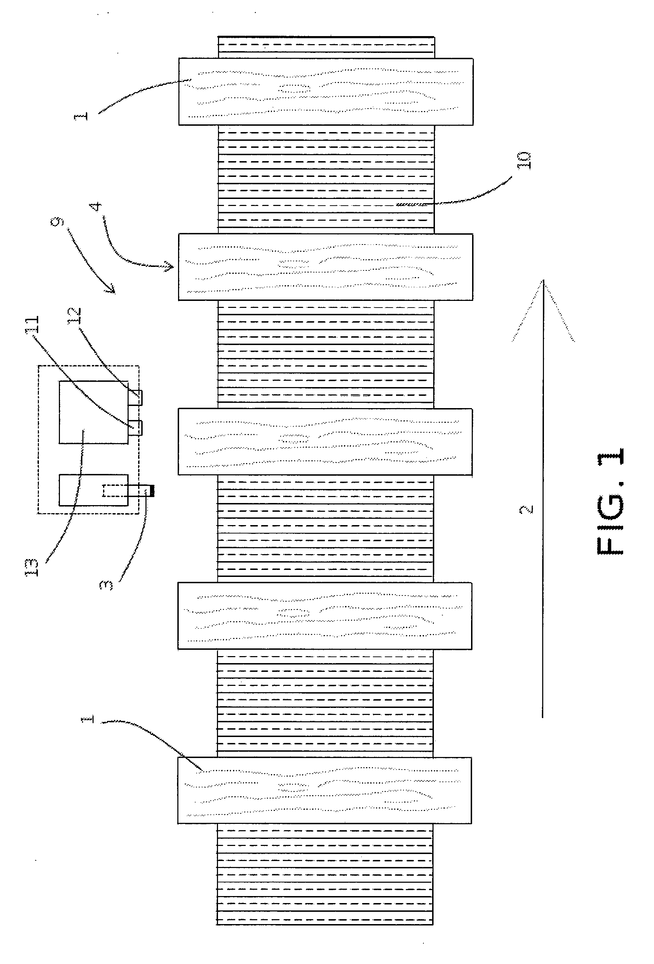

The method for determining the natural frequency of wooden planks 1 according to this invention comprises first feeding each plank 1 along a movement path, positioning each plank 1 so that its main direction of extension is transversal to the movement path. That situation is schematically illustrated in Figure 1, which shows a plurality of planks 1 being fed along a straight movement path (indicated by the arrow 2), arranged with their main direction of extension perpendicular to the movement path.

Moreover, to prevent the various planks 1 from interfering with each other, advantageously they are fed in such a way that they are distanced from each other.

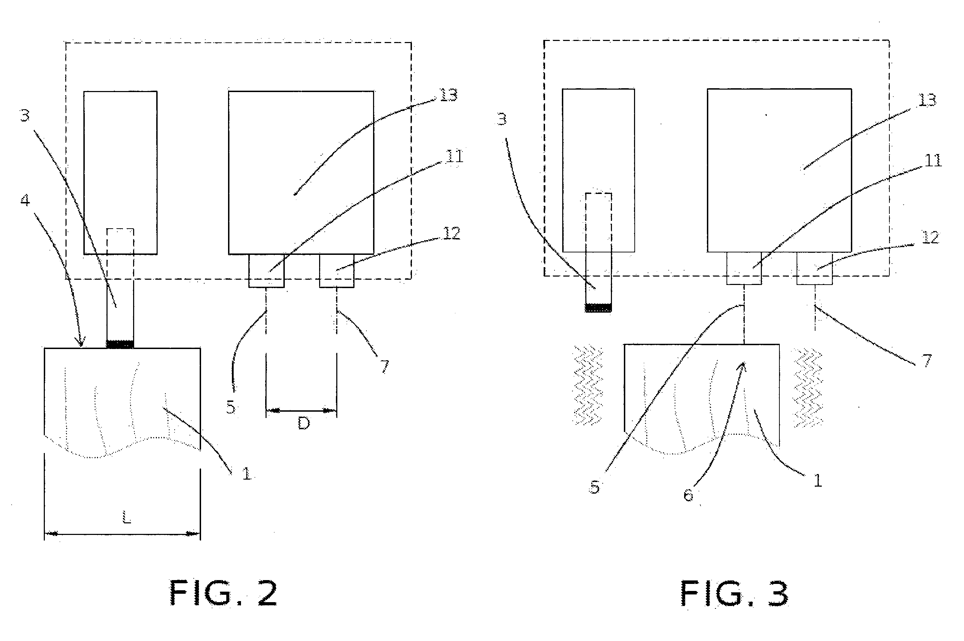

The method then comprises inducing a vibration in each plank 1, preferably without interrupting or slowing the movement of the plank 1 along the movement path (in Figures 3 to 5 the vibration is represented by the zigzag lines alongside the plank 1). That step is schematically illustrated in Figure 2, which shows the moment when a hammer 3, able to move transversally to the movement path, strikes one end 4 of the plank 1. The structure of the hammer 3, which allows it to be operated at high speeds which do not affect plank 1 feed, is known and therefore is not described in detail herein.

Then, similarly to the prior art methods, the method according to this invention comprises, during plank 1 feed along the movement path, and after a vibration has been induced in it, detection by means of a laser reading, of a firsts signal indicating the plank 1 vibration. In particular, this is done by examining the plank 1 in a first detection direction 5 which is transversal to the movement path (advantageously perpendicular to it) at a first detection position 6 (Figure 3). The laser technique for detection of the plank 1 vibration is known and therefore is not described in detail herein.

Also similarly to the prior art methods, the method according to this invention then comprises a step of determining the natural frequency by processing the first signal.

However, the method according to this invention differs from the prior art methods in the fact that it does not use only the first signal to determine the natural frequency.

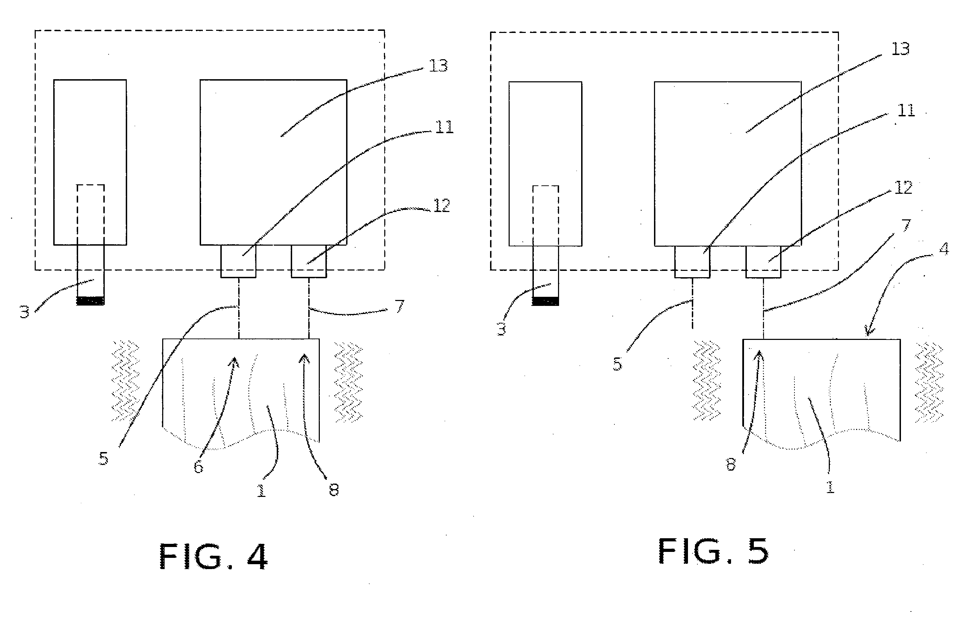

According to this invention, as the plank 1 is fed along the movement path, a laser reading is also used to detect at least one second signal indicating the plank 1 vibration (Figure 5). In particular, the second signal is detected by examining the plank 1 in a second detection direction 7 which is transversal to the movement path (advantageously perpendicular to it), and at a second detection position 8 which is distanced from the first detection position 6 in a direction parallel with the movement path.

Moreover, the natural frequency of the plank 1 is determined by processing a combination of the first and second signals. Moreover, according to requirements, the natural frequency may be determined by first determining its higher harmonics.

Also, in the preferred embodiment illustrated in the accompanying drawings, the step of detecting the second signal is carried out at least for a predetermined time simultaneously with the step of detecting the first signal. In this way, the combination of the first and second signals is made easier thanks to the fact that, for the entire time during which the readings overlap, the two signals are representative of the same phenomenon and can therefore be combined with simple time overlapping.

In the preferred embodiment, the step of determining the natural frequency is carried out after having obtained at least implicitly a third signal equal to the time overlap of the various signals detected.

It should be noticed that the third signal is obtained at least implicitly in the sense that, depending on the implementing methods, it may be effectively generated (explicit determination), or simply the algorithms for calculating the frequency are run by entering as the data to be processed the data relating to both of the readings (implicit determination). Therefore, thanks to this invention, the overall time for detection of the plank 1 vibration is equal to the sum of the individual detection times from which if necessary the overlap time for two readings must be subtracted.

The effect achieved by this invention is therefore that of virtually extending the width L of the surface of the plank 1 in such a way that the vibration can be observed for the time required by the calculation algorithms even in the case of movement speeds that are higher than those currently allowed by the prior art apparatuses. The virtual width obtained is equal to the product of the plank 1 movement speed along the path multiplied by the overall detection time.

When the movement speeds become even higher, according to this invention the step of detecting the second signal may also be repeated in a sequence a plurality of times at a plurality of separate second detection positions 8 which are located in sequence along the movement path. Similarly to the simpler case described above, each second detection position 8 is distanced from the detection position 6, 8 immediately upstream relative to the movement path (the upstream position may be either the first detection position 6 or another second detection position 8). However, advantageously, the step of detecting the second signal in each detection position 6, 8 is carried out at least for a predetermined time simultaneously with the step of detecting the signal at the detection position 6, 8 located immediately upstream.

In this more complex embodiment the method then comprises the step of determining the natural frequency of the plank 1 by using the methods described above to process a combination of the first signal with the plurality of all second signals.

Again, in particular, the natural frequency may be determined after having at least implicitly obtained a third signal equal to the time overlap of the various signals detected (using methods like those described above relative to just two readings).

This invention also relates to an apparatus 9 able to implement the method described above.

First, the apparatus 9 comprises means 10 for moving a plank 1 along a movement path which is transversal to the main direction of extension of the plank 1. In Figure 1, the movement means 10 are a conveyor. Moreover, advantageously, at least at the detection positions 6, 8, the movement means 10 are made in such a way that they do not affect the plank 1 vibration (for example, in the known way, therefore not described in detail, they may comprise an elastic belt).

The apparatus 9 also comprises means for inducing a vibration in the wooden plank 1 which in the accompanying drawings is a hammer 3 that can be operated parallel with the main direction of extension of the planks and therefore perpendicularly to the movement path formed by the movement means 10. At least a first laser detector 11 is mounted close to the movement means 10 at a first detection position 6. In accordance with what is described above, the first detector 11 has a first detection direction 5 extending transversally relative to the movement path. The first detector 11 is designed to detect the vibration of the wooden plank 1 and to generate a first signal representing said vibration.

Moreover, in accordance with this invention the apparatus 9 comprises at least one second laser detector 12 also mounted close to the movement means 10 and having a second detection direction 7 extending transversally relative to the movement path (advantageously perpendicular to it). The second detector 12 can also detect the vibration of the wooden plank 1 and generate a second signal representing said vibration. Moreover, it is located at a second detection position 8 such that the second detection direction 7 is distanced from the first detection direction 5, with reference to a direction parallel with the movement path.

Advantageously, the second detector 12 may have the same structure as the first detector 11.

The first detector 11 and the second detector 12 are connected to a processing unit 13 able to receive the first signal and the second signal and to process them. In particular, the processing unit 13 is programmed to determine the natural frequency of the plank 1 based on processing of a combination of the first signal and the second signal in accordance with the methods described above. Again, the natural frequency may be determined by first determining its higher harmonics.

However, in the preferred embodiment, the distance D between the first detection direction 5 and the second detection direction 7 is selected in such a way that it is less than the size of the plank 1 to be examined, measured parallel with the movement path (that is to say, less than the width L of the plank 1, where the width L corresponds to the plank 1 intermediate direction of extension). In an embodiment not illustrated, the apparatus 9 also comprises means for varying the distance between the first detector 11 and the second detector 12 (and therefore, between the detection directions 5, 7). Moreover, in a more complex embodiment, also not illustrated (which corresponds to the more complex embodiment described above relative to the method), the apparatus 9 comprises a plurality of second laser detectors 12 which are distributed along the movement path at a plurality of respective second detection positions 8. Each second detection position 8 is distanced from the detection position 6, 8 immediately upstream along the movement path (a position which may be, depending on the different cases, the first detection position 6 or another second detection position 8).

In this case, the processing unit 13 is connected to all of the second detectors 12 for receiving the second signals and is programmed to determine the natural frequency of the plank 1 based on processing of a combination of the first signal and the second signals.

Moreover, in this case too, advantageously the distance D between each second detection direction 7 and the detection direction (first 5 or second 7) of the detector 11,12 located immediately upstream of it, is less than the size of the plank 1 to be examined measured parallel with the movement path. Like in the case of only two detectors 11, 12, when there are more, the apparatus 9 may comprise means for varying the distance between each detector 11, 12 and those closest to it, in such a way that the distance between each pair of adjacent detectors 11, 12 is less than the minimum width L of the planks 1 to be examined.

Finally, advantageously the processing unit 13 is programmed to obtain a third signal by overlapping the times for the various signals detected and to determine the natural frequency of the plank 1 by processing the third signal obtained in that way.

This invention brings important advantages.

The method and the apparatus in accordance with this invention allow detection of the natural frequency of wooden planks being fed in such a way that they are positioned transversally to the direction of movement, whatever the feed speed.

It should also be noticed that this invention is relatively easy to produce and that even the cost linked to implementing the invention is not very high.

## Claims

1. A method for determining the natural frequency of wooden planks comprising the operating steps of: feeding each plank (1) along a movement path with the plank main direction of extension positioned transversally to the movement path; inducing a vibration in each plank (1); during feeding,
using a laser reading to detect a first signal indicating the plank (1) vibration,
examining the plank (1) in a first detection direction (5) which is transversal to the movement path at a first detection position (6); and determining the natural frequency by processing the first signal; **characterised in that** it also comprises the operating steps of: during feeding, using a laser reading to detect at least one second signal indicating the plank (1) vibration,
examining the plank (1) in a second detection direction (7) which is transversal to the movement path, at a second detection position (8) which is distanced from the first detection position (6) in a direction parallel with the movement path; and determining the natural frequency of the plank (1) by processing a combination **with time overlapping** of the first signal and the second signal.

2. The method according to claim 1, **characterised in that** the step of detecting the second signal is carried out at least for a predetermined time simultaneously with the step of detecting the first signal.

3. The method according to either of the foregoing claims, **characterised in that**, during plank (1) feed, the step of detecting the second signal is repeated a plurality of times at a plurality of separate second detection positions (8) located in sequence along the movement path and each distanced from the one upstream relative to the movement path, and also being **characterised in that** the step of determining the natural frequency of the plank (1) is carried out by processing a combination of the first signal and the plurality of second signals.

4. The method according to claim 3, **characterised in that** the step of detecting the second signal in each detection position (6), (8) is carried out at least for a predetermined time simultaneously with the step of detecting the signal at the detection position (6), (8) located immediately upstream.

5. The method according to any of the foregoing claims, **characterised in that** the step of determining the natural frequency is carried out after having obtained at least implicitly a third signal equal to the time overlap of the various signals detected.

6. An apparatus for determining the natural frequency of wooden planks (1) comprising: means (10) for moving a plank (1) along a movement path which is transversal to a main direction of extension of the wooden plank (1); means for inducing a vibration in the wooden plank (1); at least a first laser detector (11) mounted close to the movement means (10), at a first detection position (6), and having a first detection direction (5) positioned transversally relative to the movement path; the first detector (11) being able to detect the vibration of the wooden plank (1) and to generate a first signal representing said vibration; and a processing unit (13) connected to the first detector (11) for receiving the first signal and programmed to determine the natural frequency of the plank (1) based on processing of the first signal; **characterised in that** it also comprises at least one second laser detector (12) mounted close to the movement means (10) and having a second detection direction (7) positioned transversally relative to the movement path, the second detector (12) being able to detect the vibration of the wooden plank (1) and to generate a second signal representing said vibration, and the second detector (12) being located at a second detection position (8) such that the second detection direction (7) is distanced from the first detection direction (5) in a direction parallel with the movement path, and also being **characterised in that** the processing unit (13) is connected to the second detector (12) for receiving the second signal and is programmed to determine the natural frequency of the plank (1) based on processing of a combination with time overlapping of the first signal and the second signal.

7. The apparatus according to claim 6, **characterised in that** the distance D between the first detection direction and the second detection direction (7) is less than the size of the plank (1) to be examined measured parallel with the movement path.

8. The apparatus according to claim 7, **characterised in that** it comprises means for varying the distance between the first detector (11) and the second detector (12).

9. The apparatus according to any of the claims from 6 to 8, **characterised in that** it comprises a plurality of second laser detectors (12) which are distributed along the movement path at a plurality of respective second detection positions (8) each distanced from the detection position (6), (8) immediately upstream along the movement path, and also being **characterised in that** the processing unit (13) is connected to the second detectors (12) for receiving the second signals and is programmed for determining the natural frequency of the plank (1) based on processing of a combination of the first signal and the second signals.

10. The apparatus according to claim 9, **characterised in that** the distance D between each detection direction and the detection direction of the detector located immediately upstream of it is less than the size of the plank (1) to be examined measured parallel with the movement path.

11. The apparatus according to claim 10, **characterised in that** it comprises means for varying the distance between each detector and those closest to it.

12. The apparatus according to any of the claims from 6 to 11, **characterised in that** the processing unit (13) is programmed to obtain a third signal by overlapping the times for the various signals detected by the detectors and to determine the natural frequency by processing the third signal.

## Patentansprüche

1. Methode zur Bestimmung der Eigenfrequenz von Holzbrettern, enthaltend die folgenden Vorgehensschritte: Zuführung eines jeden Brettes (1) entlang einer Vorlaufbahn, mit der Hauptausdehnungsrichtung des Brettes quer zu der Vorlaufbahn angeordnet; Eingabe einer Vibration in jedes Brett (1); während des Vorlaufs Verwendung eines Laserfühlers, um ein erstes, eine Vibration in dem Brett (1) anzeigendes Signal zu erfassen, wobei das Brett (1) in einer ersten Abtastrichtung (5) quer zu der Vorlaufbahn untersucht wird, was in einer ersten Abtastposition (6) erfolgt; und die Bestimmung der Eigenfrequenz durch die Verarbeitung des ersten Signals: **dadurch gekennzeichnet, dass** sie ebenfalls die folgenden Vorgehensschritte enthält: während des Vorlaufs die Verwendung eines Laserfühlers, um wenigstens ein zweites Signal zu erfassen, das die Vibration des Brettes (1) anzeigt, wobei das Brett (1) in einer zweiten Abtastrichtung (7) quer zu der Vorlaufbahn in einer zweiten Abtastposition (8) untersucht wird, die einen Abstand von der ersten Abtastposition (6) in einer Richtung parallel zu der Vorlaufbahn hat; und die Bestimmung der Eigenfrequenz des Brettes (1) durch die Verarbeitung einer Kombination mit Zeitüberlagerung des ersten Signals und des zweiten Signals.

2. Methode nach Patentanspruch 1, **dadurch gekennzeichnet, dass** der Schritt des Erfassens des zweiten Signals wenigstens für eine bestimmte Dauer gleichzeitig mit dem Schritt des Erfassens des ersten Signals ausgeführt wird.

3. Methode nach einem jeden der vorstehenden Patentansprüche, **dadurch gekennzeichnet, dass** während des Vorlaufs des Brettes (1) der Schritt des Erfassens des zweiten Signals so viele Male wiederholt wird wie es die Anzahl der getrennten zweiten Abtastpositionen (8) ist, aufeinanderfolgend entlang der Vorlaufbahn und jede mit einem Abstand von der im Verhältnis zu der Vorlaufbahn stromaufwärts liegenden angeordnet, und ebenfalls **dadurch gekennzeichnet, dass** der Schritt zur Bestimmung der Eigenfrequenz des Brettes (1) durch die Verarbeitung eines Kombination des ersten Signals und der Anzahl von zweiten Signalen erfolgt.

4. Methode nach Patentanspruch 3, **dadurch gekennzeichnet, dass** der Schritt des Erfassens des zweiten Signals in jeder Abtastposition (6), (8) wenigstens für eine bestimmte Dauer gleichzeitig mit dem Schritt des Erfassens des Signals an der Abtastposition (6), (8) durchgeführt wird, die sich unmittelbar stromaufwärts befindet.

5. Methode nach einem jeden der vorstehenden Patentansprüche, **dadurch gekennzeichnet, dass** der Schritt zur Bestimmung der Eigenfrequenz ausgeführt wird, nachdem man wenigstens implizit ein drittes Signal erhalten hat, das der Zeitüberlagerung der verschiedenen erfassten Signale entspricht.

6. Vorrichtung zur Bestimmung der Eigenfrequenz von Holzbrettern (1), enthaltend: Mittel (10) zum Vorschieben eines Brettes (1) entlang einer Vorlaufbahn, welche quer zu einer Hauptausdehnungsrichtung des Holzbrettes (1) verläuft; Mittel zum Eingeben einer Vibration in das Holzbrett(1); wenigstens einen ersten Laserfühler (11), montiert dicht an den Vorschubmitteln (10) an einer ersten Abtastposition (6) und mit einer ersten Abtastrichtung (5), vorgesehen quer im Verhältnis zu der Vorlaufbahn; wobei der erste Fühler (11) in der Lage ist, die Vibration des Holzbrettes (1) zu erfassen und ein erstes Signal zu erzeugen, das die genannte Vibration darstellt; und eine an den ersten Fühler (11) angeschlossene Verarbeitungseinheit (13) zum Empfangen des ersten Signals und programmiert, um die Eigenfrequenz des Brettes (1) zu bestimmen, basierend auf der Verarbeitung des ersten Signals; **dadurch gekennzeichnet, dass** sie ebenfalls wenigstens einen zweiten Laserfühler (12) enthält, montiert dicht an den Vorschubmitteln (10) und mit einer zweiten Abtastrichtung (7), vorgesehen quer im Verhältnis zu der Vorlaufbahn, wobei der zweite Fühler (12) in der Lage ist, die Vibration des Holzbrettes (1) zu erfassen und ein zweites Signal zu erzeugen, das die genannte Vibration darstellt, und wobei der zweite Fühler (12) in einer zweiten Abtastposition (8) angeordnet ist, und zwar solche, dass die zweite Abtastrichtung (7) von der ersten Abtastrichtung (5) in einer Richtung parallel zu der Vorlaufbahn einen Abstand hat, und ebenfalls **dadurch gekennzeichnet, dass** die Verarbeitungseinheit (13) zum Empfangen des zweiten Signals an den zweiten Fühler (12) angeschlossen und so programmiert ist, um die Eigenfrequenz des Brettes (1) zu bestimmen, basierend auf der Verarbeitung einer Kombination mit Zeitüberlagerung des ersten Signals und des zweiten Signals.

7. Vorrichtung nach Patentanspruch 6, **dadurch gekennzeichnet, dass** der Abstand D zwischen der ersten Abtastrichtung und der zweiten Abtastrichtung (7) geringer ist als die Abmessung des zu untersuchenden Brettes (1), gemessen parallel zu der Vorlaufbahn.

8. Vorrichtung nach Patentanspruch 7, **dadurch gekennzeichnet, dass** sie Mittel zum Verändern des Abstandes zwischen dem ersten Fühler (11) und dem zweiten Fühler (12) enthält.

9. Vorrichtung nach einem jeden der vorstehenden Patentansprüche von 6 bis 8, **dadurch gekennzeichnet, dass** sie eine Anzahl von zweiten Laserfühlern (12) enthält, welche entlang der Vorlaufbahn verteilt sind, und zwar an einer Anzahl von jeweiligen zweiten Abtastpositionen (8), jede mit einem Abstand von der Abtastposition (6), (8) unmittelbar stromaufwärts entlang der Vorlaufbahn, und ebenfalls **dadurch gekennzeichnet, dass** die Verarbeitungseinheit (13) zum Empfangen der zweiten Signale an die zweiten Fühler (12) angeschlossen und so programmiert ist, um die Eigenfrequenz des Brettes (1) zu bestimmen, basierend auf der Verarbeitung einer Kombination des ersten Signals und der zweiten Signale.

10. Vorrichtung nach Patentanspruch 9, **dadurch gekennzeichnet, dass** der Abstand D zwischen jeder Abtastrichtung und der Abtastrichtung des unmittelbar stromaufwärts derselben angeordneten Fühlers geringer ist als die Abmessung des zu untersuchenden Brettes (1), gemessen parallel zu der Vorlaufbahn.

11. Vorrichtung nach Patentanspruch 10, **dadurch gekennzeichnet, dass** sie Mittel zum Verändern des Abstandes zwischen jedem Fühler und dem diesem am nächsten liegenden enthält.

12. Vorrichtung nach einem jeden der vorstehenden Patentansprüche von 6 bis 11, **dadurch gekennzeichnet, dass** die Verarbeitungseinheit (13) programmiert ist, um ein drittes Signal durch Zeitüberlagerung der verschiedenen durch die Fühler erfassten Signale zu empfangen und die Eigenfrequenz durch die Verarbeitung des dritten Signals zu bestimmen.

## Revendications

1. Une méthode pour déterminer la fréquence naturelle de planches de bois, comprenant les phases opérationnelles consistant à : faire avancer chaque planche (1) le long d'un parcours de transport avec une direction d'extension principale de ladite planche disposée transversalement par rapport audit parcours de transport ; induire une vibration dans chaque planche (1) ; pendant l'avance, exploiter une lecture laser pour détecter un premier signal indiquant la vibration de la planche (1), examiner la planche (1) dans une première direction de détection (5) qui est transversale au parcours de transport au niveau d'une première position de détection (6) ;
et déterminer la fréquence naturelle en traitant le premier signal ; **caractérisée en ce qu'**elle comprend aussi les phases opérationnelles consistant à : pendant l'avance, exploiter une lecture laser pour détecter au moins un deuxième signal indiquant la vibration de la planche (1), examiner la planche (1) dans une deuxième direction de détection (7) qui est transversale au parcours de transport, au niveau d'une deuxième position de détection (8) qui est espacée de la première position de détection (6) dans une direction parallèle audit parcours de transport ; et déterminer la fréquence naturelle de la planche (1) en traitant une combinaison avec superposition temporelle du premier signal et du deuxième signal.

2. La méthode selon la revendication 1, **caractérisée en ce que** la phase de détection du deuxième signal est effectuée, au moins pendant un temps prédéfini, en même temps que la phase de détection du premier signal.

3. La méthode selon l'une ou l'autre des revendications précédentes, **caractérisée en ce que**, durant l'avance de la planche (1), la phase de détection du deuxième signal est répétée une pluralité de fois au niveau d'une pluralité de deuxièmes positions de détection (8) distinctes situées en séquence le long du parcours de transport et espacées chacune de la position en amont par rapport audit parcours de transport, et étant aussi **caractérisée en ce que** la phase de détermination de la fréquence naturelle de la planche (1) est effectuée en traitant une combinaison du premier signal et de la pluralité de deuxièmes signaux.

4. La méthode selon la revendication 3, **caractérisée en ce que** la phase de détection du deuxième signal dans chaque position de détection (6), (8) est effectuée, au moins pendant un temps prédéfini, en même temps que la phase de détection du signal au niveau de la position de détection (6), (8) située immédiatement en amont.

5. La méthode selon l'une quelconque des revendications précédentes, **caractérisée en ce que** la phase de détermination de la fréquence naturelle est effectuée après avoir obtenu au moins implicitement un troisième signal égal à la superposition temporelle des divers signaux détectés.

6. Un appareil pour déterminer la fréquence naturelle de planches de bois (1) comprenant : des moyens (10) pour transporter une planche (1) le long d'un parcours de transport qui est transversal à une direction d'extension principale de ladite planche de bois (1) ; des moyens pour induire une vibration dans la planche de bois (1) ; au moins un premier détecteur laser (11) monté à proximité des moyens de transport (10), au niveau d'une première position de détection (6), et ayant une première direction de détection (5) disposée transversalement par rapport au parcours de transport ; le premier détecteur (11) étant destiné à détecter la vibration de la planche de bois (1) et à générer un premier signal représentant ladite vibration ; et une unité de traitement (13) reliée au premier détecteur (11) pour recevoir le premier signal et programmée pour déterminer la fréquence naturelle de la planche (1) sur la base d'un traitement dudit premier signal ; **caractérisé en ce qu'**il comprend aussi au moins un deuxième détecteur laser (12) monté à proximité des moyens de transport (10) et ayant une deuxième direction de détection (7) disposée transversalement par rapport au parcours de transport, le deuxième détecteur (12) étant destiné à détecter la vibration de la planche de bois (1) et à générer un deuxième signal représentant ladite vibration, et le deuxième détecteur (12) étant situé au niveau d'une deuxième position de détection (8) qui est telle que la deuxième direction de détection (7) est espacée de la première direction de détection (5) dans une direction parallèle au parcours de transport, et étant aussi **caractérisé en ce que** l'unité de traitement (13) est reliée au deuxième détecteur (12) pour recevoir le deuxième signal et est programmée pour déterminer la fréquence naturelle de la planche (1) sur la base d'un traitement d'une combinaison avec superposition temporelle du premier signal et du deuxième signal.

7. L'appareil selon la revendication 6, **caractérisé en ce que** la distance D entre la première direction de détection et la deuxième direction de détection (7) est inférieure à la taille de la planche (1) à examiner mesurée parallèlement au parcours de transport.

8. L'appareil selon la revendication 7, **caractérisé en ce qu'**il comprend des moyens pour faire varier la distance entre le premier détecteur (11) et le deuxième détecteur (12).

9. L'appareil selon l'une quelconque des revendications de 6 à 8, **caractérisé en ce qu'**il comprend une pluralité de deuxièmes détecteurs lasers (12) qui sont répartis le long du parcours de transport au niveau d'une pluralité de deuxièmes positions de détection (8) respectives espacées, chacune, de la position de détection (6), (8) immédiatement en amont le long du parcours de transport, et étant aussi **caractérisé en ce que** l'unité de traitement (13) est reliée aux deuxièmes détecteurs (12) pour recevoir les deuxièmes signaux et est programmée pour déterminer la fréquence naturelle de la planche (1) sur la base d'un traitement d'une combinaison du premier signal et des deuxièmes signaux.

10. L'appareil selon la revendication 9, **caractérisé en ce que** la distance D entre chaque direction de détection et la direction de détection du détecteur situé immédiatement en amont de celle-ci est inférieure à la taille de la planche (1) à examiner mesurée parallèlement au parcours de transport.

11. L'appareil selon la revendication 10, **caractérisé en ce qu'**il comprend des moyens pour faire varier la distance entre chaque détecteur et ceux qui lui sont le plus proches.

12. L'appareil selon l'une quelconque des revendications de 6 à 11, **caractérisé en ce que** l'unité de traitement (13) est programmée pour obtenir un troisième signal par le biais d'une superposition des temps des divers signaux détectés par les détecteurs et pour déterminer la fréquence naturelle par le biais d'un traitement dudit troisième signal.
